# EUROPEAN PATENT APPLICATION

(11) **EP 3 824 811 A1**
(43) Date of publication of application: **26.05.2021**
(21) Application number: 20203988.9
(22) Date of filing: 27.10.2020
(51) Int. Cl.: A61B 5/1477, A61B 5/145, G01N 33/66

(54) **NON-INVASIVE MEASUREMENT DEVICE AND METHOD**

(30) Priority: 22.11.2019 TR 201918293
(71) Applicant: Arçelik Anonim Sirketi, 34445 Istanbul (TR)
(72) Inventor: CAKIN, ILGAZ, 34445 Istanbul (TR)

(57) **Abstract**

The present invention relates to a device for and method of non-invasive analyte measurement over the skin. The non-invasive measurement device comprises a release chamber for accommodating gas to be released from the device, a suction chamber for accommodating gas withdrawn into the device, at least one opening for releasing and withdrawing gas, suction means for withdrawing the released gas into the suction chamber, measurement means for measuring a property of the withdrawn gas, and a processing unit for detecting a level of analyte.

## Description

The present invention relates to non-invasive measurement of analytes, such as glucose. The invention relates particularly to a device for and a method of non-invasive analyte measurement over the skin.

Identifying and understanding risk factors associated with many health problems or diseases require measurement of certain analyte levels in body fluids. A typical example is diabetes where monitoring the glucose levels is of utmost importance for following the development of the disease and timely medical intervention.

Almost all conventional glucose level measurement approaches are invasive. These approaches include, even if at micro scale, some sort of cutting or tearing a hole through the body, typically on the skin. Due to contamination risk and need for sterilisation following the test, these techniques are not conveniently applicable by patients at home or non-clinical environment.

One alternative approach is analyte measurement based on external irradiation and measurement of resulting flux. This approach requires wave transmission of high frequencies to minimize the influence on skin and additional sensors to monitor other bodily parameters affecting the accuracy of results. Some other techniques involve transcutaneous harvesting via interstitial fluid extraction from the skin and making measurements directly on the extracted fluid.

In the state of the art, United States patent application US2004106163 discloses a method for measuring blood glucose levels through the skin. The method comprises measuring the fluorescence spectrum emitted by a composition in the skin.

United States patent application US20110288389 discloses techniques for determining oxygen in a subject. In embodiments, an article in the form of a patch is administered to the skin of the subject to determine the oxygen level.

Korean patent application KR20180036681 discloses blood glucose measurement method relying on transcutaneous extraction of tissue fluid. Blood glucose level is determined by measuring the impedance of the tissue fluid.

The aim of the present invention is the realization of a non-invasive measurement device and method wherein the level of an analyte in a subject's body, for instance blood glucose level, can conveniently be measured on the skin. It is also aimed to provide a non-invasive measurement device and method wherein the measurement can be realized without cutting the skin or extracting body fluid. Another purpose of the invention is to realize a non-invasive measurement device and method wherein skin trauma, pain, and blood waste is eliminated or minimised. A related purpose is to realize a measurement device that is portable, simple and easy to use.

The aim of the present invention is achieved by the measurement device explicated in claim 1 and the method explicated in claim 15. Further achievements have been attained by the subject-matters respectively defined in the dependent claims.

The non-invasive measurement device for detecting a level of analyte comprises a release chamber for accommodating gas to be released from the device, a suction chamber for accommodating gas withdrawn into the device, at least one opening for releasing the gas in the release chamber out of the device and for withdrawing gas from outside the device into the suction chamber, suction means for withdrawing the released gas into the suction chamber, measurement means for measuring a property of the withdrawn gas, and a processing unit. The processing unit is arranged to determine, based on the measured property, a rate at which the released gas has been withdrawn back into the device, and detect a level of analyte based on said rate.

In a preferred embodiment, the gas to be released comprises molecules selectively binding to the analyte. The gas is preferably delivered with high pressure onto a subject's skin. The gas may be a pressurised gas and/or pumped out of the device via additional pumping means. Similarly, the gas is preferably withdrawn from the same area of the subject's skin and the withdrawn gas comprises, at least partially, the gas initially stored in the release chamber and delivered to the skin. Since the gas in the release chamber comprises molecules which can selectively bind to the measured analyte, after application to the skin the gas interacts with the analyte. Some molecules of the gas are bound to the analyte in body fluid, for example the interstitial fluid, and retained in the body fluid. As a result, the amount of gas withdrawn back into the device gives indication of the amount of analyte in the body fluid.

In an embodiment, the release chamber is arranged to accommodate a replaceable tube for storing the gas to be released. The gas to be released may be stored directly in the release chamber or placed therein within the replaceable tube.

The suction means may be a suction pump.

The measured property of the withdrawn gas is preferably the electrical conductivity. For this purpose, the measurement means may comprise anode and cathode elements, such as rods. The rods are preferably in electrical connection with the gas in the suction chamber. The measurement means may further comprise a voltmeter which may read the voltage between the anode and cathode to determine the conductivity of the gas in the suction chamber.

In embodiments, the measurement device further comprises an outlet channel between the release chamber and the opening; and/or an inlet channel between the suction chamber and the opening.

The measurement device may further comprise means to regulate flow of gas between the opening and each one of the release and suction chambers. The device may comprise at least one gas valve for selectively controlling flow of gas through the outlet channel and the inlet channel. The device may further comprise a control means, such as a control unit, for controlling the at least one gas valve. The control unit may be arranged to control the at least one gas valve, upon receiving a release signal, to open the outlet channel such that the gas in the release chamber is released from the opening. The control unit may further be arranged to control the at least one gas valve to open the inlet channel such that gas is withdrawn into the suction chamber. In a preferred embodiment, the control unit controls the at least one gas valve to open the inlet channel after a predetermined time from opening the outlet channel. Said predetermined time may be between 20 and 80 milliseconds, for example 50 milliseconds. The control unit may also be arranged to activate the suction means to withdraw gas into the suction chamber, preferably substantially at the same time as the inlet channel is opened.

The measurement device may further comprise means for outputting the detected level of analyte.

According to another aspect of the invention, there is provided a non-invasive method of detecting a level of analyte by using the measurement device explicated in the foregoing description. The method comprises: placing the device onto skin of a patient whose level of analyte is to be determined, such that the opening is pressed onto the skin; sending a release signal to the device; releasing, upon receipt of the release signal, the gas in the release chamber onto the skin, wherein the gas comprises molecules selectively binding to the analyte; withdrawing gas into the suction chamber; measuring a property of the gas in the suction chamber; determining, based on the measured property, a rate at which the released gas has been withdrawn back into the device; and detecting a level of analyte based on said rate.

The non-invasive measurement device and method realized in order to attain the aim of the present invention are illustrated in the attached figures, where:
Figure 1 is a schematic illustration of the measurement device according to an embodiment;
Figure 2 is a flow diagram of the non-invasive measurement method according to an embodiment.

The elements illustrated in the figures are numbered as follows:
1. Measurement device
2. Release chamber
3. Suction chamber
4. Opening
5. Suction means
6. Detecting means
7. Outlet channel
8. Inlet channel
9. Gas valve

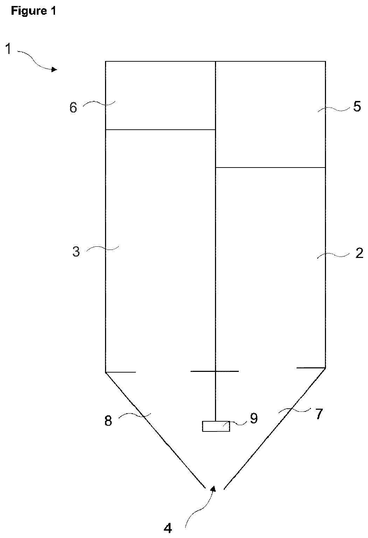

The non-invasive measurement device (1) comprises a release chamber (2) for accommodating gas to be released from the device (1), a suction chamber (3) for accommodating gas withdrawn into the device (1), at least one opening (4) for releasing the gas out of the device (1) and for withdrawing gas from outside the device (1) into the suction chamber (3), suction means (5) for withdrawing the released gas into the suction chamber (3), and measurement means (6) for measuring a property of the withdrawn gas. (Figure 1)

In a preferred embodiment, the gas accommodated in and to be released from the release chamber (2) is a gas comprising molecules which selectively bind to the analyte to measured. The gas is preferably a non-toxic, lipid soluble gas which can pass from pores of the skin. For example, the gas can be a boronic acid modified to pass through the cell membrane.

The gas is preferably applied onto subject's skin via the opening (4) such that the gas can interact with the analyte in the body fluid around the skin. With the help of suction means (5), at least part of the released gas, which have interacted with the analyte is withdrawn back into the device (1), into the suction chamber (3). The analyte may be glucose and the body fluid may be the interstitial fluid. The suction means (5) may comprise a suction pump.

The device (1) may further comprise a processing unit arranged to determine, based on the measured property, a rate at which the released gas has been withdrawn back into the device (1). The processing unit may in turn detect a level of analyte in the body fluid which has interacted with the released gas, based on said rate.

In preferred embodiments, the measurement device (1) is a portable, handheld device. The measurement device (1) preferably has a substantially tubular form, for example in the shape of a pen. The form of the device (1) may be held and operated with one hand, for example by pressing the opening (4) onto a fingertip of the other hand.

In operation, measurement device (1) is electrically powered via an internal or external power source. The measurement (1) device may comprise at least one of a battery, a power unit, a charging unit, and power and/or data connection ports. The device may further comprise control means, such as buttons, to control operation of the device. Operations such as turning on/off the device (1), starting and stopping measurement process, etc. may be realized by using the control means.

The gas is preferably pressurised gas so that permeation of the gas released from the release chamber (2) into the body fluid is facilitated. The gas may be provided directly in the release chamber (2) with high pressure. The release chamber (2) may comprise an inlet for filling pressurised gas therein. In an embodiment, the gas may be placed in the release chamber (2) in a pressurised tube. The tube may be replaceable and/or disposable. The release chamber (2) may be arranged to accommodate the high-pressure gas within or without the tube. The release chamber (2) may comprise a lid providing access to the release chamber (2) for placing and removing the tube. The pressurised gas preferably has a pressure between 130-300 psi, for example 200 psi.

The release chamber (2) and/or the tube is arranged to store a predetermined amount of gas. The amount is preferably restricted by the storage volume of the release chamber (2) and/or the tube. This is the initial amount of gas released onto the skin. In preferred embodiments, the initial amount is known by the processing unit and the rate at which the released gas has been withdrawn back into the device (1) is determined with respect to this initial amount.

The suction chamber (3) may comprise means to evacuate the withdrawn gas from the suction chamber (3). In this way, it is ensured that the withdrawn gas whose property has been measured is discharged from the suction chamber (3) and a new measurement can be made with new gas for that specific measurement. The means to evacuate the suction chamber (3) may be a release valve, which may be controlled manually or automatically by the control unit.

According to at least one embodiment, a common opening (4) is used for both release and withdrawal of the gas. In this embodiment, the opening (4) may be connected to the release chamber (2) and to the suction chamber (3) via an outlet channel (7) and an inlet channel (8) respectively. In other embodiments, the measurement device (1) may comprise more than one openings (4), comprising at least one opening for release and at least one opening (4) for withdrawal of gas. The openings (4) are preferably arranged side by side and/or in substantially the same region of the device, allowing gas to be released to and withdrawn from the same area of the skin. The size and shape of the at least one opening (4) is suitable for being pressed against the skin of the subject, for example at a fingertip of one hand.

In an embodiment, the measured property of the gas in the suction chamber (3) is its electrical conductivity. The detecting means (6) may comprise anode and cathode elements, such as rods, which preferably have electrical contact with the inner volume of the suction chamber (3). The detecting means (6) may further comprise a voltmeter. The detecting means (6) may additionally or alternatively comprise a multimeter to measure the conductivity of the gas in the suction chamber (3).

The measurement device (1) may further comprise means for opening and closing outlet of the release chamber (2) and inlet of the suction chamber (3), for example at least one gas valve (9). The at least one gas valve (9) may be arranged to selectively control flow of gas through the outlet channel (7) and the inlet channel (8).

The measurement device (1) may comprise a control unit for controlling the at least one gas valve (9). The control unit may be arranged to control the gas valve (9) to automatically open and close the outlet channel (7) and the inlet channel (8) in a pre-programmed manner in order to realize the measurement process, for example according to the method of the present invention. The control unit may comprise any suitable means to open and close the at least one gas valve (9), such as actuators.

In a preferred embodiment, the control unit is arranged to receive a release signal, and upon receipt of the release signal, control the gas valve (9) to open the outlet channel (7). When the outlet channel (7) is opened, the gas in the release chamber (2) is released from the opening (4). The release of the gas from the release chamber (3) may happen merely due to pressure difference between the chamber and the pressure outside the measurement device (1) or with additional help of a pumping means.

The control unit may be further arranged to control the gas valve (9) to open the inlet channel (9), preferably after a predetermined time from opening the outlet channel (7), so that gas is withdrawn into the suction chamber (3). The predetermined time may be set in advance by the manufacturer of the measurement device (1) and/or may be calibrated by a user of the device (1).

The predetermined time should be long enough to let the released gas interact with (e.g. molecularly bind to) the analyte in the body fluid, but not too long to let significant amount of gas disperse away from the vicinity of the opening (4). For example, oxygen molecules can permeate into a cell in about 20 milliseconds. Accordingly, the predetermined time may be more than 20 milliseconds. In preferred embodiments, the predetermined time is approximately 50 milliseconds.

The percentage of gas that will mix into air within said predetermined time can be known, or otherwise be calculated in advance. This value may be obtained from experimental data. In order to detect the amount of gas bound to the analyte, and therefore the level of analyte therefrom, the measurement device (1) is preferably calibrated to take account of the initial amount of gas in the release chamber (2) and the amount of gas that mixes into air during the predetermined time. The processing unit may detect the level of analyte accordingly.

The control unit may also be arranged to activate the suction means (5), preferably substantially at the same time as opening the inlet channel (8). Activating the suction means (5) facilitates withdrawal of gas into the suction chamber (3), increasing the amount of gas released from the release chamber (2) being withdrawn back into the measurement device (1).

The measurement device (1) may comprise means for outputting the detected level of analyte. Means for outputting may be arranged to output the detected level audibly, visually and/or haptically. The measurement device (1) may further comprise transmitting means to transmit at least one of the operating data, measurement data, and detected analyte level to an external device.

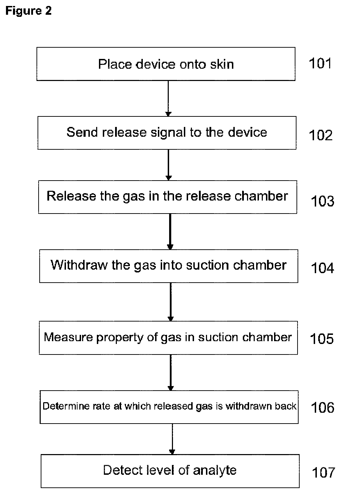

The present invention further extends to a method of non-invasively detecting a level of analyte in a body fluid, by using the measurement device (1). (Figure 2)

The method comprises placing (101) the device (1) onto skin of a patient whose level of analyte is to be determined, such that the opening (4) is pressed onto the skin. In a preferred embodiment, the detection is made around skin on the hand of the subject, by pressing the opening (4) against a fingertip, for example of an index finger. The method further comprises sending (102) a release signal to the measurement device (1). Sending (102) the release signal may comprise pressing a button on the measurement device (1). Upon receipt of the release signal, the gas in the release chamber (2), which comprises molecules that can bind to the analyte in the body fluid, is automatically released (103) onto the skin. In the example where the measurement device (1) is pressed onto a fingertip, the body fluid concerned may be the interstitial fluid surrounding the body cells at the aimed part of the skin. The method comprises, after releasing (103) the gas, withdrawing (104) the gas into the suction chamber (3). Preferably, the withdrawing (104) is automatically started upon determining that a predetermined time passed after the release of the gas. Withdrawing (104) may be facilitated by activating the suction means (5). Gas molecules which have not been bound to the analyte in the body fluid are thus withdrawn from under the skin into the suction chamber (3).

As a next step, a property of the gas in the suction chamber (3) is measured (105) and based on the measured property, a rate at which the released gas has been withdrawn back into the suction chamber (3) of the device (1) is determined (106). Based on the determined rate, level of the analyte is detected (107).

The measured property is a property of the gas that changes with the amount of gas withdrawn in the suction chamber (3). In preferred embodiments the measured property is the electrical conductivity of the gas. Electrical conductivity will be roughly proportional to the amount of the released gas withdrawn back into the suction chamber (3). Hence, higher the amount of analyte in body fluid will bind to and retain a higher amount of released gas in body fluid, which in turn will decrease the amount of gas withdrawn back into the measurement device (1) and therefore the conductivity in the suction chamber (3). Accordingly, the measured property of the gas gives an indication of the rate of released gas withdrawn back into the suction chamber (3). Given that this rate is changes inversely with the amount of released gas retained by the analytes, a level of the analyte can be detected.

For an improved accuracy of the detected level, the measurement device (1) should be maintained in the same position with respect to the skin during application of the method.

The method may further comprise at least one of: storing the detected level in a memory, outputting the detected level through outputting means, and transmitting the detected level to another device via any suitable means.

In embodiments where a tube comprising the release gas is used, the method may additionally comprise placing the tube into the release chamber (3) and removing the empty tube from the release chamber (3).

In view of technical features and details explained above, the present invention proposes a measurement device (1) and methods for detecting a level of an analyte in a body fluid using said device (1), wherein the measurement is made in a non-invasive manner and without extracting the body fluid out of the body. According to the invention, a gas which selectively binds to the analyte whose level is to be detected is applied to the skin of a subject and immediately withdrawn back into the measurement device (1). Based on the amount of the gas not bound to the analyte in a body fluid around the target region of the skin and withdrawn back into the suction chamber (3) of the device (1), the level of analyte of interest can be indirectly detected. With use of the measurement device (1) and method, a level of analyte in a body fluid, for example glucose level in interstitial fluid can be easily detected by simply pressing the device (1) on a subject's skin for a few seconds.

## Claims

1. A non-invasive measurement device (1) for detecting a level of analyte, comprising: a release chamber (2) for accommodating gas to be released from the device (1), wherein the gas to be released comprises molecules selectively binding to the analyte; a suction chamber (3) for accommodating gas withdrawn into the device (1); at least one opening (4) for releasing the gas in the release chamber (2) out of the device (1) and for withdrawing gas from outside the device (1) into the suction chamber (3); suction means (5) for withdrawing the released gas into the suction chamber (3); measurement means (6) for measuring a property of the withdrawn gas; and a processing unit, wherein the processing unit is arranged to determine, based on the measured property, a rate at which the released gas has been withdrawn back into the device (1), and detect a level of analyte based on said rate.

2. The measurement device (1) according to claim 1, wherein the device (1) comprises a release chamber (2) for accommodating a replaceable tube for storing the gas to be released.

3. The measurement device (1) according to claim 1 or 2, wherein the suction means (5) is a suction pump.

4. The measurement device (1) according to any one of preceding claims, wherein the measured property is electrical conductivity.

5. The measurement device (1) according to claim 4, wherein the measurement means (6) comprises anode and cathode rods.

6. The measurement device (1) according to claim 5, wherein the measurement means further comprises a voltmeter.

7. The measurement device (1) according to any one of preceding claims, wherein the measurement device (1) further comprises an outlet channel (7) between the release chamber (2) and the opening (4); and an inlet channel (8) between the suction chamber (3) and the opening (4).

8. The measurement device (1) according to claim 7, wherein the measurement device (1) further comprises at least one gas valve (9) for selectively controlling flow of gas through the outlet channel (7) and the inlet channel (8).

9. The measurement device (1) according to claim 8, wherein the measurement device (1) further comprises a control unit for controlling the at least one gas valve (9).

10. The measurement device (1) according to claim 9, wherein the control unit is arranged to, upon receiving a release signal, control the at least one gas valve (9) to open the outlet channel (7) such that the gas in the release chamber (2) is released from the opening (4).

11. The measurement device (1) according to claim 10, wherein the control unit is further arranged to, after a predetermined time from opening the outlet channel (7), controlling the at least one gas valve (9) to open the inlet channel (8) such that gas is withdrawn into the suction chamber (3).

12. The measurement device (1) according to claim 11, wherein the predetermined time is 50 milliseconds.

13. The measurement device (1) according to claim 11 or 12, wherein the control unit is further arranged to, substantially at the same time as the inlet channel (8) is opened, activate the suction means (5) to withdraw gas into the suction chamber (3).

14. The measurement device (1) according to any one of preceding claims, wherein the measurement device (1) further comprises means for outputting the detected level of analyte.

15. A non-invasive method of detecting a level of analyte by using the measurement device (1) according to any one of preceding claims, the method comprising:
placing (101) the device (1) onto skin of a patient whose level of analyte is to be determined, such that the opening (4) is pressed onto the skin,
sending (102) a release signal to the device (1),
releasing (103), upon receipt of the release signal, the gas in the release chamber (2) onto the skin, wherein the gas comprises molecules selectively binding to the analyte,
withdrawing (104) gas, preferably upon determining that a predetermined time passed after the release of the gas, into the suction chamber (3),
measuring (105) a property of the gas in the suction chamber (3),
determining (106), based on the measured property, a rate at which the released gas has been withdrawn back into the device (1), and
detecting (107) a level of analyte based on said rate.
